# EUROPEAN PATENT APPLICATION

(11) **EP 3 805 370 A1**
(43) Date of publication of application: **14.04.2021**
(21) Application number: 19812038.8
(22) Date of filing: 30.05.2019
(51) Int. Cl.: C12N 5/0775, C12N 9/22, C12N 15/10, C12N 15/113, A61K 35/28, A61P 1/02, A61P 19/10, A61P 29/00

(54) **HLA GENE-DELETED, HUMAN INDUCED PLURIPOTENT STEM CELL-DERIVED MESENCHYMAL STEM CELL AND PREPARATION METHOD THEREFOR**

(30) Priority: 30.05.2018 KR 20180062166
(71) Applicant: Kangstem Biotech Co., Ltd., Seoul 06179 (KR)
(72) Inventor: KANG, Kyung-Sun, Seoul 06338 (KR); SEO, Kwang Won, Suwon-si Gyeonggi-do 16300 (KR); AHN, Hee-Jin, Seoul 02598 (KR); KWON, Daekee, Seoul 02598 (KR); HAN, Mi-Jung, Seoul 02725 (KR)
(74) Representative: Zwicker, Jörk
(86) International application number: PCT/KR2019/006536
(87) International publication number: WO 2019/231266

(57) **Abstract**

Provided are a method of preparing induced pluripotent stem cells (iPSCs) and embryonic stem cells (ESCs), both having a reduced expression level of HLA, which is a cause of immune rejection upon transplantation of cells, tissues, or organs, a method of preparing mesenchymal stem cells (MSCs) by using a novel differentiation method of iPSCs, and a cell therapy product using the iPSCs, ESCs, and/or MSCs. The HLA gene-deleted MSCs prepared in the present invention may be used to prepare various types of cells that do not cause immune rejection, and may be used for therapeutic purposes in the future.

<110>     Kangstem Biotech CO., LTD.

<120>     Mesenchymal stem cells with engineered HLA gene from induced
          pluripotent stem cells and a method for producing them

<130>     OPA19137

<150>     KR 10-2018-0062166
<151>     2018-05-30

<160>     9

<170>     KoPatentIn 3.0

<210>     1
<211>     20
<212>     DNA
<213>     Artificial Sequence

<220>
<223>     HLA-A


<400>     1
gagccccgct tcatcgccgt                                                   20


<210>     2
<211>     20
<212>     DNA
<213>     Artificial Sequence

<220>
<223>     HLA-B


<400>     2
ccttgccgtc gtaggcgtac                                                   20


<210>     3
<211>     20
<212>     DNA
<213>     Artificial Sequence

<220>
<223>     HLA-C


<400>     3
gtcactcacc gtcctcgctc                                                   20


<210>     4
<211>     22
<212>     DNA
<213>     Artificial Sequence

<220>
<223>     HLA-A_F


<400>     4

tttctccctc tcccaaccta tg                                                22

<210>    5
<211>    20
<212>    DNA
<213>    Artificial Sequence

<220>
<223>    HLA-A_R

<400>    5
cgttcagggc gatgtaatcc                                                   20

<210>    6
<211>    20
<212>    DNA
<213>    Artificial Sequence

<220>
<223>    HLA-B_F

<400>    6
cccacttccc actcccattg                                                   20

<210>    7
<211>    22
<212>    DNA
<213>    Artificial Sequence

<220>
<223>    HLA-B_R

<400>    7
cttcccgttc tccaggtatc tg                                                22

<210>    8
<211>    20
<212>    DNA
<213>    Artificial Sequence

<220>
<223>    HLA-C_F

<400>    8
caatcagcgt ctccgcagtc                                                   20

<210>    9
<211>    20
<212>    DNA
<213>    Artificial Sequence

<220>
<223>    HLA-C_R

<400>    9
ctccaggtag gctctcagct                                                    20

## Description

### [Technical Field]

The present invention relates to a method of preparing induced pluripotent stem cells (iPSCs) and embryonic stem cells (ESCs), both the cells having a reduced expression level of HLA, which is a cause of immune rejection upon transplantation of cells, tissues, or organs, a method of preparing mesenchymal stem cells (MSCs) by using a novel differentiation method of iPSCs, and a cell therapy product using the iPSCs and MSCs.

### [Background Art]

Major histocompatibility antigen (MHC) is known as human leukocyte antigen (human HLA) and is expressed in most cells and tissues. HLA mainly has six gene loci of A, B, C, DR, DQ, and DP, each of which is composed of dozens of different types (alleles) of complex combinations, and there are tens of thousands of combinations. HLA has an important immune mechanism in the human body, and the principal function thereof is to present antigens for self-recognition. If cells or tissues are transplanted from another person (allogeneic transplantation), HLA acts as the most important antigen and is recognized by immune cells, such as T cells, *etc.,* and engraftment of the transplant does not occur.

Meanwhile, as demonstrated in recent treatment of various diseases, adult stem cells that may differentiate into various cells are believed to have tremendous potential in the field of therapeutics. For example, a technology for preparing induced pluripotent stem cells (iPSCs), which enables reprogramming of various types of cells isolated from the human body into pluripotent cells similar to embryonic stem cells (Cell, 126: 663-676, 2006; Cell, 131:1-12, 2007), provides a novel strategy for patient-specific differentiation of clinically available lineage-specific cells such as endothelial progenitor cells.

However, despite the potential of stem cells, stem cells whose HLA type does not match that of a recipient may induce immune rejection during transplantation. Thus, HLA antigens are a major obstacle to clinical application of stem cells. Since there are close to tens of thousands of combinations of HLA alleles, it is a very difficult problem to obtain a natural HLA homozygous cell line (Rim et al., J Tissue Eng Regen Med, 2017). Genetically engineered HLA molecules are considered as a preferred alternative to avoid immune rejection.

### [Disclosure]

### [Technical Problem]

The present inventors have made many efforts to develop a method of obtaining transplantation-compatible cells that do not cause an immune rejection in a recipient and a cell population including the same, and as result, they prepared induced pluripotent stem cells, in which alleles are knocked out by targeting a gene encoding an HLA alpha or beta subunit, and HLA class I homozygous mesenchymal stem cells differentiated from the induced pluripotent stem cells by a novel method, and they confirmed a differentiation potential of the mesenchymal stem cells, thereby completing the present invention.

### [Technical Solution]

An object of the present invention is to provide a method of preparing mesenchymal stem cells, the method including the steps of (a) reducing an expression level of HLA class I of induced pluripotent stem cells (iPSCs) using a gene scissors technology; (b) generating embryoid bodies (EBs) from the induced pluripotent stem cells of the step (a); and (c) differentiating the embryoid bodies of the step (b) into mesenchymal stem cells (MSCs).

Another object of the present invention is to provide transplantation-compatible mesenchymal stem cells prepared according to the above method.

Still another object of the present invention is to provide a pharmaceutical composition for preventing or treating any one disease selected from the group consisting of immune diseases, inflammatory diseases, neurological diseases, bone diseases, and arthropathy, the pharmaceutical composition including the transplantation-compatible mesenchymal stem cells as an active ingredient.

Still another object of the present invention is to provide a cell therapy product including the transplantation-compatible mesenchymal stem cells as an active ingredient.

Still another object of the present invention is to provide a method of preparing transplantation-compatible induced pluripotent stem cells, the method including the step of reducing an expression level of HLA class I using a gene scissors technology.

Still another object of the present invention is to provide transplantation-compatible induced pluripotent stem cells prepared according to the above method.

Still another object of the present invention is to provide a method of preparing transplantation-compatible embryonic stem cells (ESCs), the method including the step of reducing an expression level of HLA class I using a gene scissors technology.

### [Advantageous Effects]

By differentiating HLA gene-deleted iPSCs prepared in the present invention, MSCs may be prepared, which may be used to prepare various types of cells that do not cause an immune rejection, and may be used for therapeutic purposes in the future.

### [Brief Description of the Drawings]

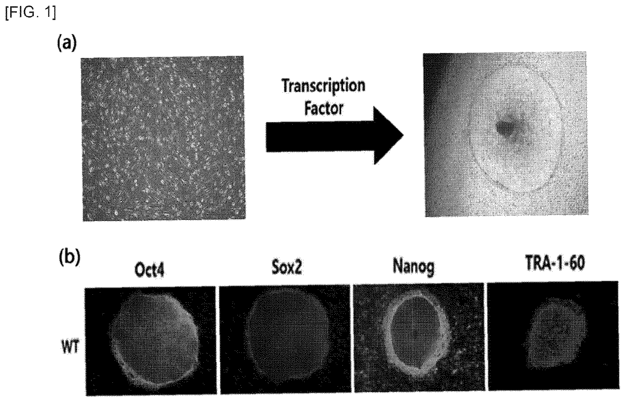

FIG. 1 shows photographs showing a preparation process of wild-type iPSCs, and protein expression of the prepared iPSCs, wherein FIG. 1A shows a microscopic image showing morphology of cells which were prepared by introducing a retrovirus expressing Yamanaka factors (Oct4, Sox2, c-Myc, and Klf-4) into human dermal fibroblasts (hDFs), in which the prepared cells exhibited induced pluripotent stem cell-specific morphology, and FIG. 1B shows a microscopic image showing expression of surface proteins (Oct4, Sox2, Nanog, and TRA-1-60) in the prepared iPSCs, visualized by immunostaining;

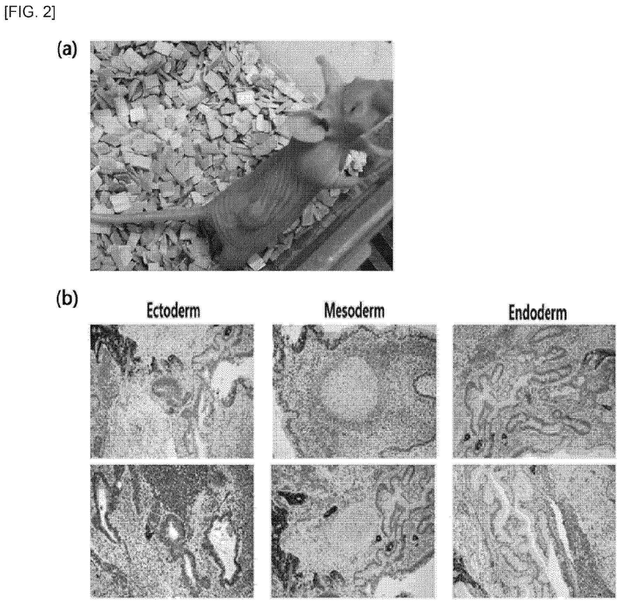

FIG. 2 shows a teratoma generated by injecting the wild-type iPSCs into a nude mouse, wherein FIG. 2A shows a photograph showing teratoma formation in the nude mouse, and FIG. 2B shows ectodermal, mesodermal, and endodermal cells observed in tissues of the formed teratoma;

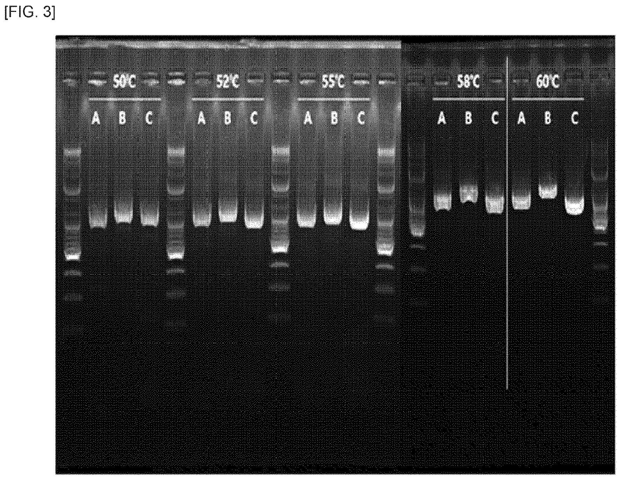

FIG. 3 shows a photograph showing the results of electrophoresis of PCR amplification products of HLA-A, HLA-B, and HLA-C loci;

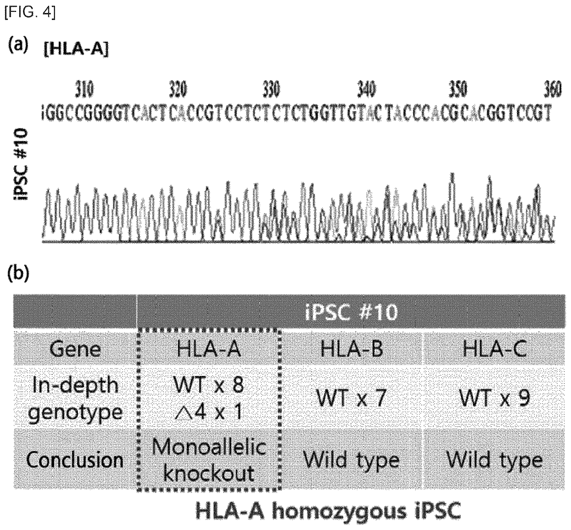

FIG. 4 shows genotyping of iPSC#10 among the formed iPSC colonies, wherein FIG. 4A shows genotyping of iPSC#10 using primers after purifying PCR products, indicating HLA-A-knockout iPSCs, in which one allele of HLA-A gene was knocked out, and FIG. 4B shows a table showing in-depth genotyping of iPSC#10, indicating HLA-A homozygous iPSCs, in which one allele of HLA-A gene, among HLA-A, HLA-B, and HLA-C belonging to HLA class I, was knocked out;

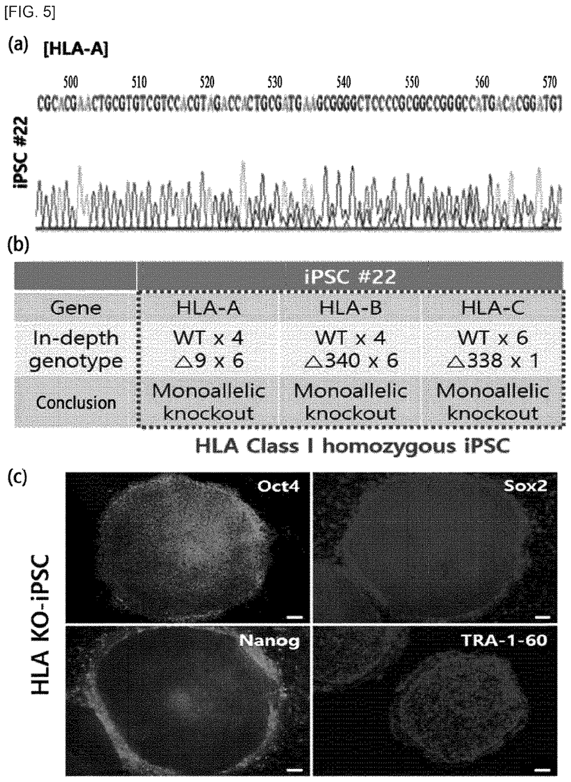

FIG. 5 shows genotyping of iPSC#22 among the formed iPSC colonies, wherein FIG. 5A shows genotyping of iPSC#22 using primers after purifying PCR products, indicating HLA-A-knockout iPSCs, in which one allele of HLA-A gene was knocked out, FIG. 5B shows a table showing in-depth genotyping of iPSC#22, indicating HLA class I homozygous iPSCs, in which each one allele of HLA-A, HLA-B, and HLA-C was knocked out, and FIG. 5C shows strong expression of Oct4, Sox2, Nanog, and TRA-1-60 proteins which are pluripotency marker proteins, visualized by immunostaining of iPSC#22;

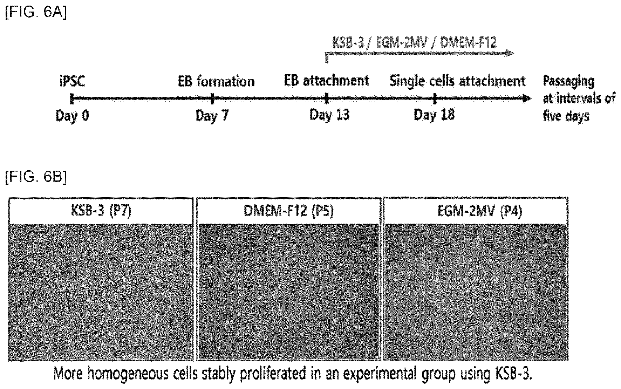

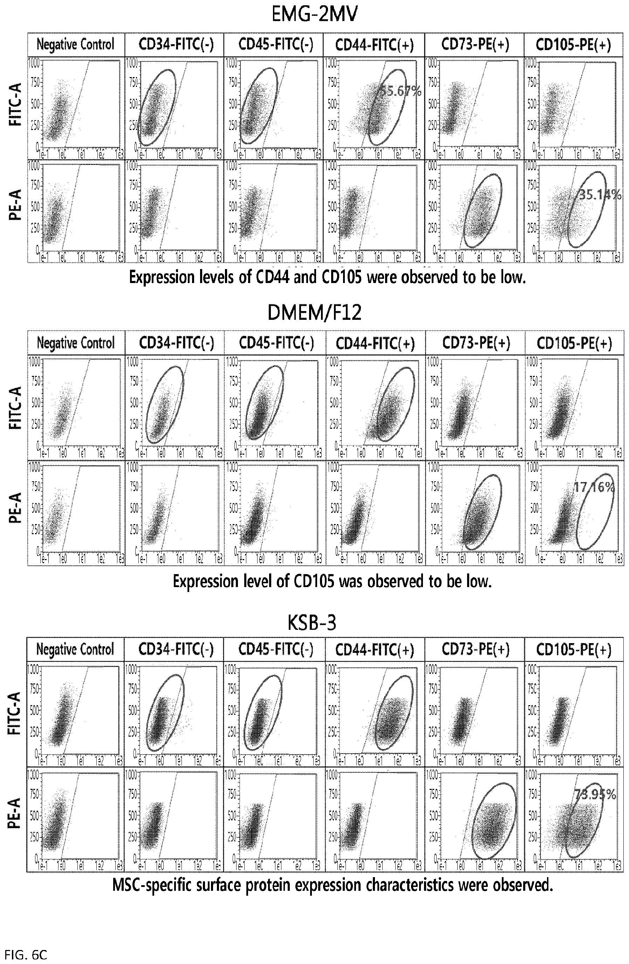

FIG. 6 shows a method of differentiating MSCs from iPSCs of Example 2-1 and the result thereof, wherein FIG. 6A shows a schematic illustration of the differentiation method, in which embryoid bodies were generated from iPSCs, and 6 days later, embryoid bodies were cultured in KSB-3, EGM-2MV, or DMEM-F12 medium, FIG. 6B shows photographs showing morphology of the cells proliferated in each medium, and FIG. 6C shows the result of FACS analysis of surface markers of the cells proliferated in each medium;

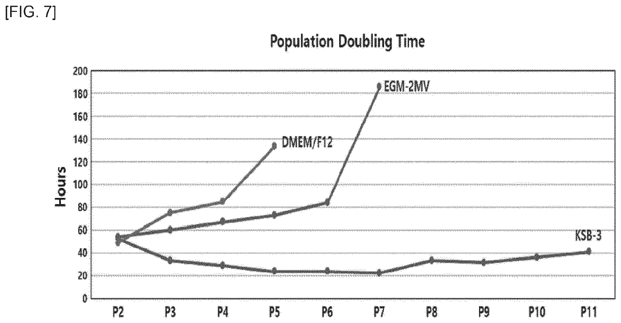

FIG. 7 shows a graph showing time changes required for cell proliferation according to the increasing passages in each culture medium;

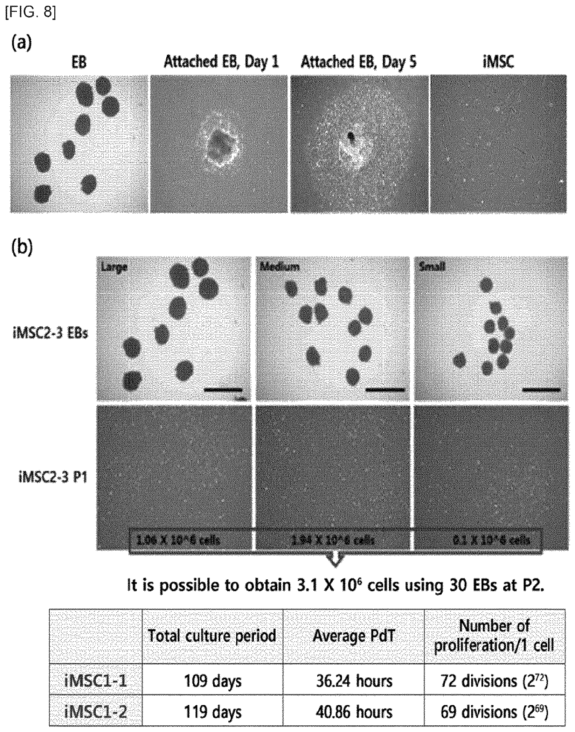

FIG. 8 shows characteristics of MSCs (iMSCs) differentiated from iPSCs, wherein FIG. 8A shows photographs showing a differentiation process of MSCs by culturing iPSCs, and FIG. 8B shows a proliferation potential of iMSCs;

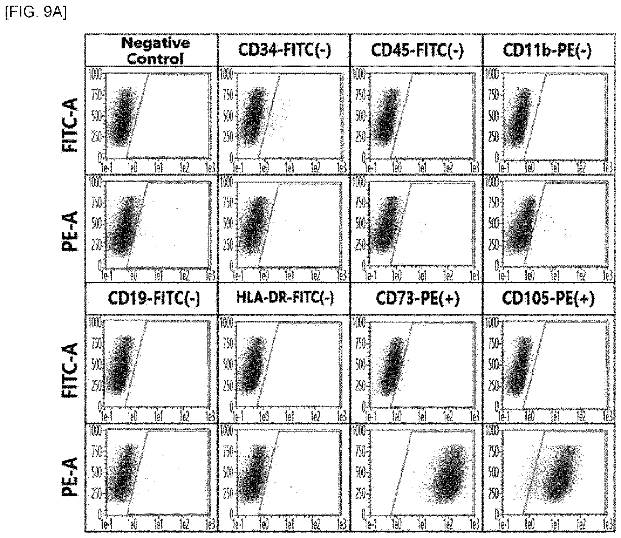

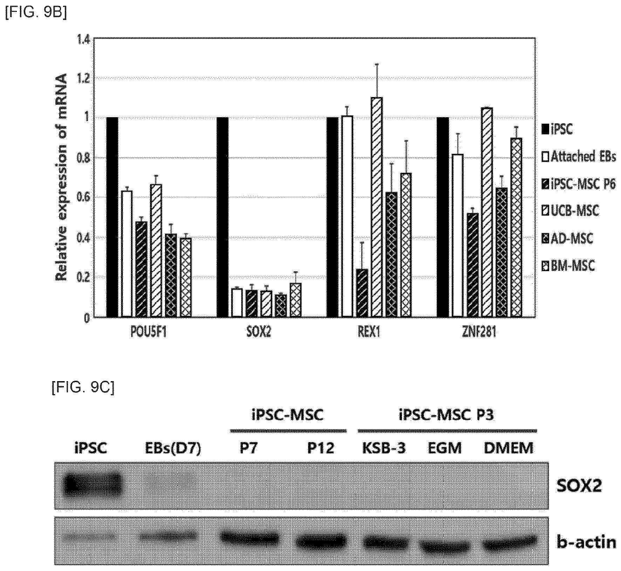

FIG. 9 shows characteristics of the prepared iMSCs, wherein FIG. 9A shows FACS analysis showing expression of surface antigens (CD11b, CD19, CD34, CD45, HLA-DR, CD73, CD105) of the prepared iMSCs, FIG. 9B shows a bar graph showing mRNA expression levels of stemness genes (POU5F1, SOX2, REX1, ZNF281) of iPSCs and iMSCs differentiated therefrom, and FIG. 9C shows western blot analysis showing expression of a pluripotency marker Sox2 in iPSCs and iMSCs differentiated therefrom;

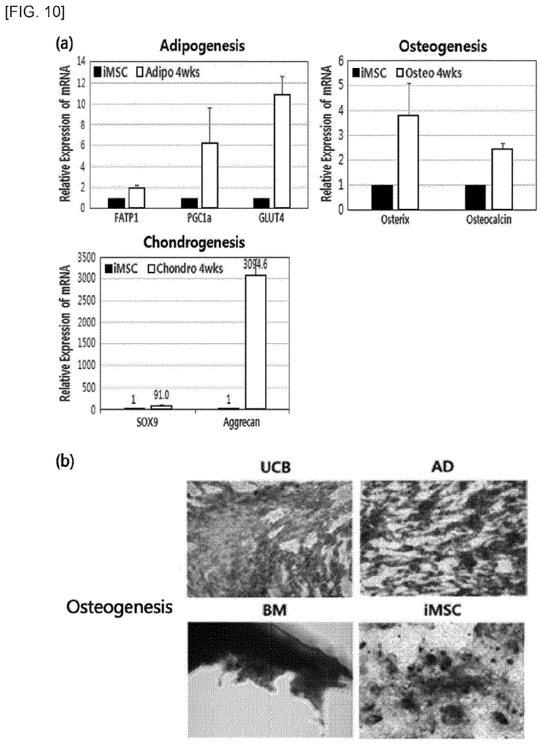

FIG. 10 shows the differentiation potential of the prepared iMSCs, wherein FIG. 10A shows a graph showing the qRT-PCR results of measuring the expression levels of genes related to adipocyte, osteocyte, and chondrocyte differentiation in the prepared iMSCs, and FIG. 10B representatively shows the differentiation potential into osteocytes (UCB represents cord blood stem cells, AD represents fat-derived stem cells, BM represents bone marrow-derived stem cells, and iMSC represents MSCs differentiated from iPSCs);

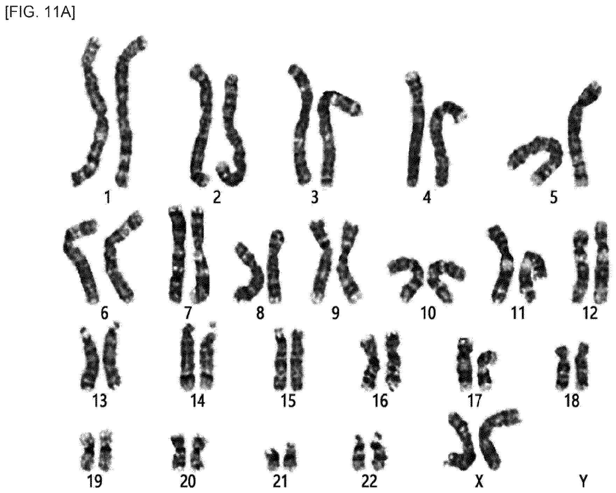

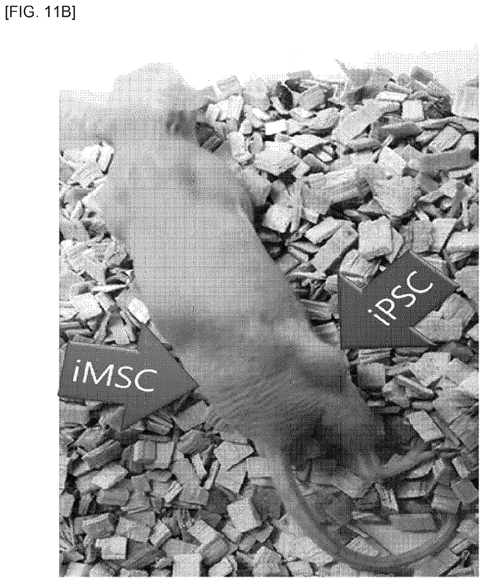

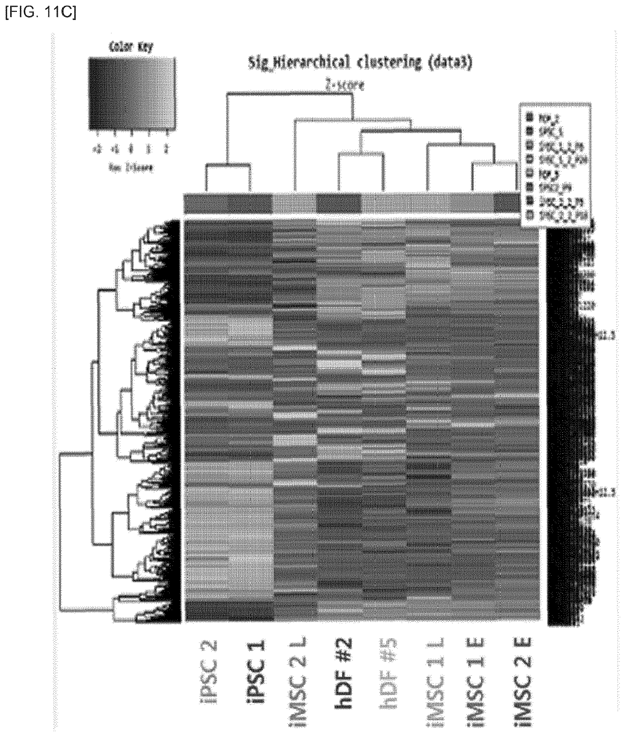

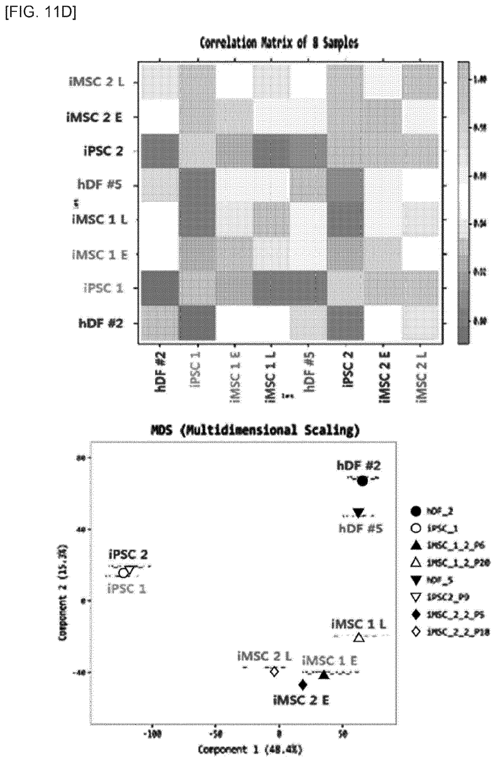

FIG. 11 shows genetic stability and hierarchical clustering of the prepared iMSCs, wherein FIG. 11A shows the result of karyotyping of the prepared iMSCs, in which the normal karyotype was observed, FIG. 11B shows *in vivo* stability of the prepared iMSCs, in which the sites where iMSCs and iPSCs were injected into an experimental mouse are indicated by arrows, a tumor was formed at the site where iPSCs were injected (right side of the mouse in FIG. 11B), whereas no tumor was formed at the site where iMSCs were injected (left side of the mouse in FIG. 11B), and FIGS. 11C and 10D show the results of performing microarray using hDFs, iPSCs, and iMSCs in order to examine hierarchical clustering of the prepared iMSCs, in which iMSCs showed hierarchical clustering separated from iPSCs while showing an expression system more similar to iPSCs than hDFs; and

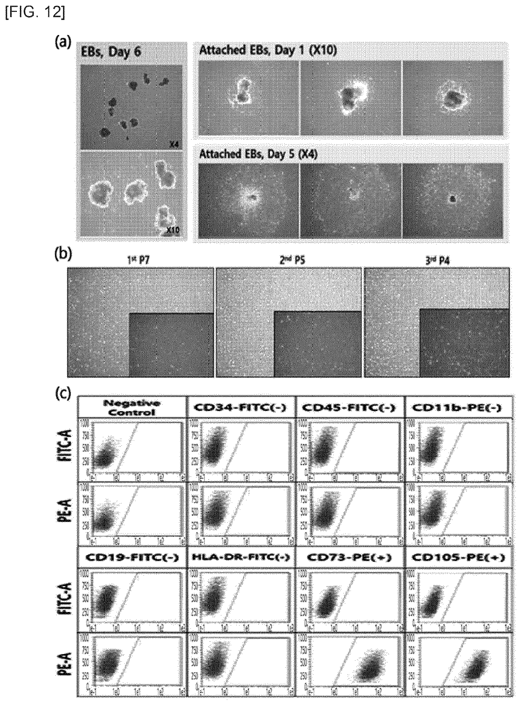

FIG. 12 shows the results of observing characteristics of HLA class I homo iMSCs derived from HLA class I homo iPSCs which were differentiated through the method of Example 2-1, wherein FIG. 12A shows photographs showing differentiation of embryoid bodies, attached embryoid bodies and cells during the differentiation process of HLA class I homo iPSCs in the same manner as in Example 2-1, FIG. 12B shows photographs showing the morphology of HLA class I homo iMSCs, in which the MSC-specific cell morphology was observed, like the existing iMSCs, and FIG. 12C shows the results of FACS analysis of examining the MSC-specific surface antigen expression of HLA class I homo iMSCs.

### [Best Mode for Carrying Out the Invention]

Each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

To achieve the above objects, an aspect of the present invention provides a method of preparing mesenchymal stem cells, the method including the steps of (a) reducing an expression level of HLA class I of induced pluripotent stem cells (iPSCs) using a gene scissors technology; (b) generating embryoid bodies (EBs) from the induced pluripotent stem cells of the step (a); and (c) differentiating the embryoid bodies of the step (b) into mesenchymal stem cells (MSCs).

In the present invention, the step (a) is a step of reducing an expression level of HLA class I of induced pluripotent stem cells (iPSCs) using a gene scissors technology.

As used herein, the term "induced pluripotent stem cells (iPSCs)" refers to cells induced from differentiated cells by an artificial reprogramming process so as to have a pluripotent differentiation potential. The artificial reprogramming process may be performed by the use of a virus-mediated vector such as retrovirus or lentivirus or a non-viral vector or by introduction of non-virus-mediated reprogramming factors using proteins, cell extracts, *etc.,* or may include a reprogramming process performed by stem cell extracts, compounds, *etc.* Specifically, the process may include a process of introducing a retrovirus expressing Oct4, Sox2, c-Myc, or Klf-4 into differentiated cells, but is not limited thereto. The induced pluripotent stem cells have similar characteristics, specifically, similar cell morphology and similar gene and protein expression patterns to those of embryonic stem cells, and for example, may strongly express proteins such as Oct4, Sox2, Nanog, TRA-1-60, *etc.,* but are not limited thereto. Further, the induced pluripotent stem cells may have pluripotency *in vitro* and *in vivo,* may form teratomas including three germ layer cells when subcutaneously injected into mice, and are capable of germline transmission of genes.

As used herein, the term "HLA (human leukocyte antigen)" is synonymous with histocompatibility antigen, and is also called major histocompatibility antigen complex (MHC), a cell surface protein. Humans are known to express six species derived from paternal genes and six species derived from maternal genes, that is, a total of six pairs. The role of HLA is to display fragments of proteins present in the cells on the surface of the cells and to enable infections or mutations that might occur in the body to be detected by the immune cells. For this reason, they are also called antigen presenting proteins (APP). If these APP are not autologous cells, they are the main targets of immune cells present in the transplant recipient's body. In general, the HLA class is used to test tissue compatibility between a donor and a recipient upon transplantation of cells, tissues, or organs. Transplantation is possible if all of HLA antigens of the donor and the recipient match. However, the total allele number of HLA classes I and II is 9719 or more, and thus it is not easy to find a fully matched donor because each person has numerous genetic polymorphisms on each HLA gene. Upon transplantation of cells, tissues, or organs, if the HLA-A gene of a donor and the HLA-A gene of a recipient do not match, the recipient's body immune cells recognize the difference and attack the donor cells, eventually leading to graft failure by immune rejection. In this regard, the present invention provides a method capable of preparing a transplantation-compatible cell, specifically, a cell in which a histocompatibility antigen is homozygous or null through gene manipulation.

As used herein, the term "transplantation-compatible" refers to a property that does not cause immune rejection during cell transplantation. By reducing expression of a gene that causes the immune rejection, cells, tissues, or organs may be allowed to have transplantation compatibility. As a non-limiting example thereof, by reducing the expression level of the HLA gene, cells, tissues, or organs may be allowed to have transplantation compatibility. Transplantation-compatible cells may include, but are not limited to, cells in which the histocompatibility antigen is homozygous or null.

The HLA may be classified into "HLA class I" and "HLA class II". HLA-A, HLA-B, and HLA-C belong to HLA class I, and HLA-DR, HLA-DP, and HLA-DQ belong to HLA class II. General somatic cells express a total of only three pairs, *i.e.,* HLA-A, HLA-B, and HLA-C belonging to HLA class I, and immune cells express a total of six pairs, the sum of HLA class I and HLA class II.

As used herein, the term "step of reducing the expression level using the gene scissors technology" means that a gene encoding the corresponding polypeptide is not expressed by knockin or knockout, or the gene expression shows a specific decrease, as compared with the natural state or the state before modification, or the functional corresponding polypeptide is not produced even if it is expressed.

The term also means that not only is the gene encoding the corresponding polypeptide completely inactivated, but also, the expression level is weakened or significantly lower than the intrinsic expression level and thus is not substantially expressed. Thus, gene inactivation may be complete (knockout) or partial (*e.g.,* a hypomorph, in which a gene exhibits less than intrinsic expression levels, or a product of a mutant gene that shows partial reduction in the activity it influences).

In addition, a decrease in the gene expression level may occur in one of the two pairs of alleles, which is "homozygous", or may occur in all of the two pairs of alleles, which is "null". That is, with respect to the decrease in the gene expression level, the term "homozygous" used herein may be interpreted as meaning that one allele is knocked out, and the term "null" may be interpreted as meaning that two alleles are knocked out. Specifically, "HLA-homo" cells prepared in one specific embodiment of the present invention mean "HLA-homozygous" cells.

Specifically, HLA inactivation of the present invention may be performed by:
1) deleting a part or the entirety of a polynucleotide encoding the protein,
2) modifying the expression control sequence for reducing the expression of the polynucleotide,
3) modifying the polynucleotide sequence on the chromosomes to weaken the activity of the protein, or
4) a combination thereof, but is not particularly limited thereto.

1) The method of deleting a part or the entirety of a polynucleotide encoding the protein may be performed by replacing the polynucleotide encoding the endogenous target protein within the chromosome with a polynucleotide having a partial deletion in the polynucleotide sequence or a marker gene using a vector for intracellular chromosomal insertion. As used herein, the term "a part" may vary depending on the kinds of polynucleotides, specifically, 1 to 300, more specifically, 1 to 100, and even more specifically 1 to 50.

Next, 2) the method of modifying the expression control sequence for reducing the expression of the polynucleotide may be, but is not particularly limited to, performed by inducing a modification on the expression control sequence through deletion, insertion, non-conservative or conservative substitution of the polynucleotide sequence, or a combination thereof to further weaken the activity of the expression control sequence, or by replacing the polynucleotide sequence with a polynucleotide sequence having a weaker activity. The expression control sequence includes a promoter, an operator sequence, a sequence encoding a ribosome-binding site, and a sequence regulating the termination of transcription and translation, but is not limited thereto.

Additionally, 3) the method of modifying a polynucleotide sequence on the chromosome may be performed by inducing a modification on the polynucleotide sequence through deletion, insertion, non-conservative or conservative substitution of the polynucleotide sequence, or a combination thereof to further weaken the activity of the protein, or by replacing the polynucleotide sequence with an improved polynucleotide sequence having a weaker activity, but is not limited thereto.

The "gene scissors technology" of the present invention may include the step of reducing the expression of HLA antigens through gene manipulation, specifically, gene deletion or gene modification, using a "gene scissors (engineered nuclease)", but is not limited thereto. The gene manipulation includes gene deletion or gene modification, and may be performed using a method called gene editing, genome editing, or genome engineering technology. The gene manipulation may be performed by knocking out or knocking in a specific gene, but is not limited thereto. The gene manipulation technique may be to use an enzyme that specifically acts on a desired sequence by including a nuclease or nickase and a guide vector, but is not limited thereto. Specifically, the gene scissors may include, for example, zinc finger nuclease (ZFN), transcription activator-like effector nuclease (TALEN), clustered regularly interspaced short palindromic repeat (CRISPR), but is not limited thereto. In addition, the target sequence may be knocked out in such a way that the gene scissors and a template DNA similar to a target sequence are simultaneously introduced into the cells, and the template DNA introduced into the target sequence is knocked in by homologous recombination.

The "zinc finger nuclease" refers to a fusion protein including a zinc finger domain and a nucleotide cleavage domain, and may include all known or commercial zinc finger nucleases. As used herein, the term "zinc finger nuclease" may be used interchangeably with "ZFN".

The "transcription activator-like effector nuclease (TALEN)" refers to an artificial restriction enzyme prepared by fusing TAL effector DNA binding domain with a DNA cleavage domain. Further, the TALEN of the present invention also includes the TAL effector DNA binding domain and the DNA cleavage domain which are linked to each other via a linker. Here, the TAL effector DNA binding domain is engineered to rapidly bind to any desired DNA sequence, and when the TAL effector DNA binding domain is combined with the DNA cleavage domain, it specifically binds to the desired DNA sequence, having an effect of cutting the desired DNA site.

When knockout or knockin is performed using the gene scissors, knockin is performed using a donor DNA together with nuclease, unlike knockout in which the donor DNA is not necessarily used, besides nuclease. The donor DNA is a DNA containing a gene to be introduced at a location on a chromosome cut by the nuclease, and may include a left flanking arm and a right flanking arm for recombination, and may selectively include a selection marker, *etc.,* but is not limited thereto.

The "RNA-guided nuclease" is a nuclease capable of recognizing and cleaving a specific location on a target genome, and specifically, refers to a nuclease with target specificity by guide RNA. The RNA-guided nuclease may be, but is not limited to, specifically, Cas protein derived from CRISPR, which is a bacterial immune system, or Cpf1 nuclease, and more specifically, CRISPR-Associated Protein 9 (Cas9) nuclease and a variant thereof, Cas9 nickase.

The "Cas protein" is a main protein component of the CRISPR/Cas system, and is a protein acting as an activated endonuclease or nickase. The Cas protein may form a complex with CRISPR RNA (crRNA) and trans-activating crRNA (tracrRNA) to exhibit activity thereof.

The Cas9 nuclease induces double strand break (DSB) by recognizing a specific nucleotide sequence in the genome of plant cells and animal cells as well as human cells. The double strand break includes generation of a blunt end and a cohesive end by cutting the double helix of DNA. DSB is efficiently repaired by homologous recombination or non-homologous end-joining (NHEJ) mechanism in cells. During this process, researchers may introduce desired mutations into target sites. The RNA-guided nuclease may be artificial or one that is engineered and not naturally occurring.

The Cas9 nickase may have D10A mutation, but is not limited thereto. Unlike Cas9 nuclease, the Cas9 nickase causes a single strand break. Therefore, two guide RNAs are required for the Cas9 nickase to work, and function as a pair. The two guide RNAs direct sequence-specific binding of the CRISPR complex with respect to each target sequence, and direct cleavage of one strand of the DNA duplex near each target sequence to induce two nicks in different DNA strands.

Information on Cas protein or gene is available at public databases such as GenBank of the National Center for Biotechnology Information (NCBI). Specifically, the Cas protein may be Cas9 protein. Further, the Cas protein may be Cas protein derived from the genus *Staphylococcus,* the genus *Streptococcus,* the genus *Neisseria,* the genus *Pasteurella,* the genus *Francisella,* or the genus *Campylobacter,* but the present invention is not limited to the examples described above.

The "Cpf1 nuclease" may be derived from *Francisella novicida,* and may form a complex with crRNA to exhibit activity thereof. Further, the structure of Cpf1 protein is different from the Cas9 protein, and it binds to short RNA. Thus, preparation thereof is easy with excellent accuracy. Information of Cpf1 protein or gene is available at a public database such as GenBank of the National Center for Biotechnology Information (NCBI). The "step of reducing an expression level of HLA class I using a gene scissors technology" of the present invention may be a step of knocking out one or more of HLA-A, HLA-B and HLA-C, specifically, a step of knocking out all of HLA-A, HLA-B and HLA-C, using a composition for knocking out HLA class I, but is not limited thereto. Further, the composition for knocking out may include a CRISPR/Cas9 system targeting HLA class I, but is not limited thereto. Specifically, the composition for knocking out HLA class I may include one or more of (a) an expression vector including an isolated polynucleotide encoding a guide RNA consisting of a nucleotide sequence of SEQ ID NO: 1 or a nucleotide sequence complementary thereto; (b) an expression vector including an isolated polynucleotide encoding a guide RNA consisting of a nucleotide sequence of SEQ ID NO: 2 or a nucleotide sequence complementary thereto; and (c) an expression vector including an isolated polynucleotide encoding a guide RNA consisting of a nucleotide sequence of SEQ ID NO: 3 or a nucleotide sequence complementary thereto, but is not limited thereto. Further, in addition to the step of reducing an expression level of HLA class I using a gene scissors technology, the step of reducing an expression level of a gene belonging to HLA class II, *i.e.,* one or more genes of HLA-DR, HLA-DP, and HLA-DQ, may be further included.

In a specific embodiment of the present invention, CRIPSR/Cas9 expression vectors including SEQ ID NO: 1 to SEQ ID NO: 3 as guide RNAs, each targeting HLA-A, HLA-B and HLA-C belonging to HLA class I, were prepared, and HLA class I was knocked out through a process of introducing all of the expression vectors.

Further, the expression vector may include a polynucleotide encoding Cas9 nuclease or Cas9 nickase. In other words, since a target DNA may be cleaved when Cas9 protein and a guide RNA co-exist, a vector, in which the polynucleotide encoding Cas9 protein is included together with the guide RNA, may be used, or a separate expression vector encoding Cas9 protein may be used. The vector may be a viral vector, a plasmid vector, or an *agrobacterium* vector, but is not limited thereto. The expression vector including the polynucleotide encoding the guide RNA and/or the polynucleotide encoding Cas9 protein may be prepared by performing a cloning method known in the art, and the method is not particularly limited thereto.

In the present invention, the step (b) is a step of generating embryoid bodies (EBs) from the induced pluripotent stem cells of the step (a).

As used herein, the term "embryoid body (EB)" refers to a cell aggregate of pluripotent stem cells, and may be used to refer to a state in which stem cells reach the early stage of differentiation after being separated from feeder cells. The step of generating embryoid bodies from stem cells may include a method of changing a composition of a stem cell medium, specifically, excluding specific components, growth factors, *etc.* from the stem cell medium, but is not limited thereto. Specifically, the stem cell culture medium may include DMEM/F12+GlutaMAX-I (Gibco), Primocin (Invivogen), Knockout SR (Gibco), basic fibroblast growth factor (R&D), *etc.,* and in the present invention, a medium prepared by excluding the basic fibroblast growth factor from the medium was used to generate embryoid bodies.

In the present invention, the step (c) is a step of differentiating the embryoid bodies of the step (b) into mesenchymal stem cells (MSCs).

As used herein, the term "mesenchymal stem cell" refers to a heterogeneous population of stem cells having self-renewal and capacity to differentiate into mesodermal lineage and different embryonic lineages such as endodermal and ectodermal lineages. The mesenchymal stem cells may be specifically an originator of cartilage, bone, fat, bone marrow stroma, muscle, nerve, skin, *etc.* In adults, the mesenchymal stem cells usually remain in the bone marrow, but may be obtained from umbilical cord, umbilical cord blood, peripheral blood, other tissues, *etc.* The mesenchymal stem cells may be obtained from induced pluripotent stem cells through differentiation, but are not limited thereto.

In one specific embodiment of the present invention, the mesenchymal stem cells were obtained by differentiating the induced pluripotent stem cells, and the obtained mesenchymal stem cells were confirmed to stably differentiate into adipocytes, chondrocytes, and osteocytes. These results confirmed that the mesenchymal stem cells of the present invention may be differentiated into cells constituting various tissues, and thus the mesenchymal stem cells of the present invention may be used to generate differentiated cells.

To differentiate mesenchymal stem cells, a KSB-3 (Kangstem biotech) medium may be used. Specifically, in a specific embodiment of the present invention, to differentiate the induced pluripotent stem cells into the mesenchymal stem cells, KSB-3 (Kangstem biotech) medium containing 10% FBS was used. After generating embryoid bodies, they are not immediately separated into single cells, but the embryoid bodies are cultured in KSB-3 (Kangstem biotech) medium containing 10% FBS for 6 days to selectively proliferate the differentiated mesenchymal stem cells in a large amount.

Further, the "differentiated cells" which may be obtained by using the mesenchymal stem cells of the present invention refer to any cells that are at the stage of differentiating into somatic cell lineages or have finally differentiated. In other words, the differentiated cells refer to cells constituting an adult, having restricted differentiation potential and self-renewal. Specifically, the differentiated cells may be somatic cells constituting cartilage, bone, fat, bone marrow stroma, muscle, nerve, skin, *etc.* of humans, but are not limited thereto.

Another aspect of the present invention provides transplantation-compatible mesenchymal stem cells prepared according to the above method. The terms "transplantation-compatible" and "mesenchymal stem cell" are the same as described above.

Still another aspect of the present invention provides a pharmaceutical composition for preventing or treating any one disease selected from the group consisting of immune diseases, inflammatory diseases, neurological diseases, bone diseases, and arthropathy, the pharmaceutical composition including, as an active ingredient, the mesenchymal stem cells prepared according to the above method.

As used herein, the term "immune disease" refers to a disease that causes problems when specific immune responses occur, and may include, but is not limited to, preferably autoimmune diseases, transplant rejection, or graft versus host disease. The autoimmune diseases may include Crohn's disease, erythema, atopy, rheumatoid arthritis, Hashimoto's thyroiditis, pernicious anemia, Addison's disease, type 1 diabetes, lupus, chronic fatigue syndrome, fibromyalgia, hypothyroidism and hyperthyroidism, scleroderma, Behçet's disease, inflammatory bowel disease, multiple sclerosis, myasthenia gravis, Ménière's syndrome, Guillain-Barré syndrome, Sjögren's syndrome, vitiligo, endometriosis, psoriasis, vitiligo, systemic scleroderma, asthma, ulcerative colitis, *etc.*

As used herein, the term "inflammatory disease" collectively refers to a disease having inflammation as a main lesion, and may include, but is not limited to, preferably edema, dermatitis, allergy, atopy, asthma, conjunctivitis, periodontitis, rhinitis, tympanitis, sore throat, tonsillitis, pneumonia, gastric ulcer, gastritis, Crohn's disease, colitis, hemorrhoids, gout, ankylosing spondylitis, rheumatic fever, lupus, fibromyalgia, psoriatic arthritis, osteoarthritis, rheumatoid arthritis, periarthritis of the shoulder, tendinitis, tenosynovitis, myositis, hepatitis, cystitis, nephritis, Sjögren's syndrome, or multiple sclerosis.

The "neurological disease" of the present invention includes a disease caused by deformation or loss of nerve cells, and specifically, may include Parkinson's disease, Alzheimer's disease, Pick's disease, Huntington's disease, amyotriophiclateral sclerosis, ischemic brain disease (stroke), demyelinating disease, multiple sclerosis, epilepsy, neurodegenerative diseases, spinal cord injury, *etc.,* but is not limited thereto.

The "bone disease" of the present invention includes all diseases that may be caused by decreased bone density, and specifically, may include osteoporosis, rheumatoid arthritis, periodontal disease, osteomalacia, osteogenesis imperfecta, osteopetrosis, osteosclerosis, Paget's disease, adynamic bone disease, metabolic bone disease, rickets, *etc.,* but is not limited thereto. Since the mesenchymal stem cells of the present invention have a bone differentiation potential, they may be administered to an individual with a bone metabolic disease, and thus may be used as a composition for osteosis.

The "arthropathy" of the present invention, also called "arthrosis" or "osteo-arthrosis", refers to a disease involving a lack of pain perception and joint abnormalities, and is classified as a neurotrophic disease of the joint. Generally, the arthropathy includes polyarthrosis, coxarthrosis, gonarthrosis, spondyloarthropathy, *etc.,* and may be accompanied by cartilage damage or cartilage dysfunction. It is known that since most arthropathy is accompanied by inflammation, it may develop into arthritis. In one specific embodiment of the present invention, the mesenchymal stem cells were confirmed to differentiate into chondrocytes, and thus the mesenchymal stem cells of the present invention may be used as a composition for preventing or treating arthropathy.

As used herein, the term "preventing" means all of the actions by which occurrence of diseases is restrained or retarded by administering the composition.

As used herein, the term "treating" means all of the actions by which diseases have improved or been modified favorably by administering the composition.

The composition may include a pharmaceutically acceptable carrier.

The "pharmaceutically acceptable carrier" may mean a carrier or diluent that does not cause irritation to an organism and does not abrogate biological activity and properties of a compound to be injected. The kind of the carrier applicable in the present invention is not particularly limited, and any carrier which is commonly used in the art and is pharmaceutically acceptable may be used. Non-limiting examples of the carrier may include a saline solution, sterile water, Ringer's solution, a buffered saline solution, an albumin injectable solution, a dextrose solution, a maltodextrin solution, glycerol, ethanol, *etc.,* which may be used alone or in a combination of two or more thereof.

The composition including the pharmaceutically acceptable carrier may have various oral or parenteral dosage forms. When formulated, it is prepared by using diluents or excipients, such as fillers, extenders, binders, wetting agents, disintegrants, surfactants, *etc.,* which are commonly used.

In detail, solid preparations for oral administration include tablets, pills, powders, granules, capsules, *etc.,* and these solid preparations are prepared by mixing the compound with at least one excipient, *e.g.,* starch, calcium carbonate, sucrose, lactose, gelatin, *etc.* Further, in addition to simple excipients, lubricants, such as magnesium stearate talc, *etc.* are also used. Liquid preparations for oral administration may include suspensions, liquids for internal use, emulsions, syrups, *etc.,* and various excipients such as wetting agents, sweeteners, fragrances, and preservatives may be included, in addition to water and liquid paraffin, which are commonly used simple diluents. Preparations for parenteral administration include sterile aqueous solutions, non-aqueous solvents, suspensions, emulsions, lyophilized preparations, and suppositories. As the non-aqueous solvents and the suspensions, propylene glycol, polyethylene glycol, vegetable oil such as olive oil, and injectable esters such as ethyl oleate may be used. As a base for suppositories, witepsol, macrogol, tween 61, cacao butter, laurin butter, glycerogelatin, *etc.* may be used.

Still another aspect of the present invention provides a cell therapy product including, as an active ingredient, the transplantation-compatible mesenchymal stem cells prepared according to the above method. The transplantation-compatible mesenchymal stem cells are the same as described above.

As used herein, the term "cell therapy product", which refers to cells and tissues prepared by isolation from an individual, culture, and specific manipulation, is a pharmaceutical drug used for the purpose of treatment, diagnosis, and prevention (U.S. FDA regulations). The term also refers to a pharmaceutical drug used for the purpose of treatment, diagnosis, and prevention through a series of actions such as proliferating and selecting living autologous, allogenic, or xenogenic cells *in vitro* or changing biological properties of the cells by different methods, in order to restore the functions of cells or tissues.

The cell therapy product of the present invention may include 1.0×10 cells to 1.0×10⁹ cells per 1 mL, but is not limited thereto. The cell therapy product of the present invention may be used without freeze-drying or may be freeze-dried for later use. When freeze-drying is needed, standard cryopreservative (*e.g*., DMSO, glycerol, and Epilife cell freeze-drying medium (Cascade Biologics)) may be added to a cell population before freeze-drying. Additionally, the cell therapy product may be administered after being formulated into a unit administration form suitable for administration into a patient's body according to a common method in the pharmaceutical field, and the formulation may include an administration dose which will be effective after single administration or several administrations. Examples of the formulations suitable for this purpose, as a formulation for parenteral administration, may be preferably injections such as injection ampoules, infusion agents such as infusion bags, and spraying agents such as aerosol formulations, *etc.* The injection ampoules may be prepared by mixing with an injection immediately prior to use. For injection solutions, physiological saline, glucose, mannitol, Ringer's solution, *etc.* may be used. Additionally, for infusion bags, materials of polyvinyl chloride or polyethylene may be used, and may be exemplified by infusion bags manufactured by Baxter, Becton Dickinson, Medcep, National Hospital Products, or Terumo.

The cell therapy product may further include one or more pharmaceutically acceptable common inert carrier, *e.g.,* a preserving agent, an analgesic, a solubilizer, a stabilizer, *etc.* for injectable formulations, and a base, an excipient, a lubricant, a preserving agent, *etc.* for topical formulations.

The cell therapy product of the present invention thus prepared may be administered by a common administration method used in the art along with other stem cells, which are used for transplantation and other uses, or in a mixed form with these stem cells, and may be directly engrafted or transplanted to the disease area, or directly transplanted or infused to the abdominal cavity of a patient in need of treatment, but is not limited thereto. Further, the administration may be administered either by a non-surgical administration method using a catheter or by a surgical administration method by injecting into or transplanting to the disease area after cutting the disease area off, but it is more preferable to administer by a non-surgical administration method using a catheter. In addition to parenteral administration according to a common method, for example, direct administration into the lesions, transplantation by infusion into the blood vessel, which is a general method for hemopoietic stem cell transplantation, is also possible.

The cell therapy product may be administered once or as several divided doses. However, it should be understood that the actual dose of active ingredients is determined considering various related factors, such as a disease to be treated, severity of the disease, administration route, a patient's body weight, age, and gender, *etc.,* and thus the administration dose should not be construed as limiting the scope of the present invention in any manner.

In one specific embodiment of the present invention, it was confirmed that the mesenchymal stem cells may differentiate into adipocytes, osteocytes, and chondrocytes. Thus, the mesenchymal stem cells of the present invention may be used as a therapeutic agent for various diseases.

Still another aspect of the present invention provides a method of preparing transplantation-compatible induced pluripotent stem cells, the method including the step of reducing the expression level of HLA class I using the gene scissors technology.

The "transplantation-compatible" and the "induced pluripotent stem cell" are the same as described above.

Specifically, the HLA class I may be one or more selected from the group consisting of HLA-A, HLA-B, and HLA-C, and the gene scissors technology may be any one of CRISPR, TALEN, and zinc finger nuclease, but is not limited thereto. In addition, the step of reducing the expression level of HLA class I using the gene scissors technology may be performed by using a composition for knocking out HLA class I, and the composition for knocking out HLA class I may include one or more selected from expression vectors including isolated polynucleotides encoding guide RNAs consisting of nucleotide sequences of SEQ ID NO: 1, SEQ ID NO: 2, and SEQ ID NO: 3 or nucleotide sequences complementary thereto. Further, the expression vector may include a polynucleotide encoding Cas9 nuclease, Cpf1 nuclease, or Cas9 nickase.

Still another aspect of the present invention provides transplantation-compatible induced pluripotent stem cells prepared by the above method. The terms "transplantation-compatible" and "induced pluripotent stem cell" are the same as described above.

Still another aspect of the present invention provides a method of preparing transplantation-compatible embryonic stem cell (ESCs), the method including the step of reducing the expression level of HLA class I using the gene scissors technology, and transplantation-compatible embryonic stem cells prepared by the preparation method.

The embryonic stem cells (ES cells) of the present invention are cells having the potential to differentiate into cells of all tissues constituting an individual, and may be used interchangeably with the term "pluripotent stem cells" or "totipotent stem cells". The embryonic stem cells may be undifferentiated cells capable of unlimited proliferation, may differentiate into all cells, and may generate germ cells, unlike adult stem cells.

The HLA class I, the gene scissors technology, and the transplantation-compatible are the same as described above.

### [Mode for Carrying Out the Invention]

Hereinafter, the present invention will be described in more detail with reference to Examples and Experimental Examples. However, these Examples and Experimental Examples are for illustrative purposes only, and the scope of the present invention is not intended to be limited by these Examples and Experimental Examples.

### Example 1: Preparation of HLA allele-knockout iPSCs using CRISPR/Cas9 system

### Example 1-1: Preparation of wild-type iPSCs and Examination of pluripotency

To prepare wild-type iPSCs, a retrovirus expressing Yamanaka factors (Oct4, Sox2, c-Myc, Klf-4) was introduced into human dermal fibroblasts (hDFs) to prepare iPSCs (FIG. 1A).

It was confirmed by immunostaining that the prepared iPSCs strongly expressed Oct4, Sox2, Nanog, and TRA-1-60 proteins, which are proteins exhibiting pluripotency (FIG. 1B), and it was also confirmed that when the prepared iPSCs were subcutaneously injected into a nude mouse, a teratoma including three germ layer cells was formed (FIGS. 2A and 2B), indicating that iPSCs have pluripotency *in vitro* as well as *in vivo.*

### Example 1-2: Preparation of target sequence (HLA class I)-specific CRISPR/Cas9

Guide RNA sequences (SEQ ID NOS: 1 to 3) capable of removing HLA-A, HLA-B, and HLA-C genes belonging to HLA class I were designed. The designed sequences are shown in Table 1 below.

**[Table 1]**

| Gene | Guide RNA sequence | SEQ ID NO: |
|---|---|---|
| HLA-A | GAGCCCCGCTTCATCGCCGT | 1 |
| HLA-B | CCTTGCCGTCGTAGGCGTAC | 2 |
| HLA-C | GTCACTCACCGTCCTCGCTC | 3 |

Each of the prepared guide RNA sequences was cloned into a pCas-guide vector to prepare three kinds of CRISPR/Cas9 vectors, each targeting HLA-A, HLA-B, or HLA-C gene.

### Example 1-3: Preparation of HLA gene-knockout iPSCs

To prepare HLA gene-knockout iPSCs, the following experiment was performed.

In detail, three kinds of CRISPR/Cas9 vectors, each capable of removing HLA-A, HLA-B, or HLA-C gene, prepared in Example 1-2 were simultaneously introduced into a single cell preparation of iPSCs by electroporation, and then cultured in a CloneR™ (STEMCELL Technologies)-supplemented iPSC medium for 10 days to 14 days. Thereafter, the formed single iPSC cell-derived colonies were separated and cultured.

Then, genomic DNAs were isolated from 24 iPSC colonies separated and cultured as above, and then HLA-A, HLA-B, and HLA-C loci were amplified by PCR using the isolated genomic DNAs and primers. The primers used in the amplification are shown in Table 2 below.

**[Table 2]**

| Gene | Sequence (5' to 3') | Size (bp) | Annealing temp (°C) | SEQ ID NO: |
|---|---|---|---|---|
| HLA-A | F-TTTCTCCCTCTCCCAACCTATG | 1,000 | 60 | 4 |
| | R-CGTTCAGGGCGATGTAATCC | | | 5 |
| HLA-B | F-CCCACTTCCCACTCCCATTG | 1,091 | 60 | 6 |
| | R-CTTCCCGTTCTCCAGGTATCTG | | | 7 |
| HLA-C | F-CAATCAGCGTCTCCGCAGTC | 1,011 | 60 | 8 |
| | R-CTCCAGGTAGGCTCTCAGCT | | | 9 |

The amplified PCR products were purified by electrophoresis (FIG. 3), and then genotypes were examined by using PCR primers. As a result, among 24 iPSC colonies, iPSC#10 was confirmed to be an HLA-A-knockout iPSC, in which one allele of HLA-A gene was knocked out (FIG. 4A). Further, the amplified PCR product was subjected to TA cloning, transformation, colony inoculation, and plasmid miniprep, and in-depth genotyping thereof was performed. As a result, iPSC#10 was confirmed to be an HLA-A homozygous iPSC, in which one of two HLA-A alleles was knocked out (FIG. 4B).

Further, among 24 iPSC colonies, iPSC#22 was confirmed to be an HLA-class I-knockout iPSC, in which one allele of HLA-class I gene was knocked out (FIG. 5A), and the amplified PCR product was subjected to TA cloning, transformation, colony inoculation, and plasmid miniprep, and in-depth genotyping thereof was performed. As a result, iPSC#22 was confirmed to be an HLA class I homozygous iPSC, in which one of two alleles of each of HLA-A, HLA-B, and HLA-C was knocked out (FIG. 5B).

It was also confirmed by immunostaining that the prepared HLA class I-knockout iPSCs strongly expressed Oct4, Sox2, Nanog, and TRA-1-60 proteins, which are proteins exhibiting pluripotency (FIG. 5C), indicating that iPSCs have pluripotency.

### Example 2: Method of inducing differentiation of mesenchymal stem cells (MSCs) from iPSCs and characterization of MSCs prepared therefrom

### Example 2-1: Induction of differentiation of MSCs from iPSCs and examination of efficiency

To differentiate iPSCs into mesenchymal stem cells (MSCs), the following experiment was performed.

In detail, the wild-type iPSCs prepared in Example 1-1 were cultured for 7 days, and then iPSCs were separated from a culture dish using a dissociation solution or mechanical subculture. The separated iPSCs were washed with PBS three times, and cultured in a petri dish available for suspension culture to induce embryoid body (EB) formation, thereby inducing primary differentiation. As a medium for the EB formation, a general iPSC medium (DMEM/F12+GlutaMAX-I (Gibco) 500 mL + Primocin (Invivogen) 1 mL + Knockout SR (Gibco) 125 mL + Basic fibroblast growth factor (R&D) 2.5 µg) was used, from which bFGF (basic fibroblast growth factor) needed to maintain undifferentiation had been removed. The medium was replaced every 2-3 days.

6 days after EB formation, EBs were attached to a culture dish which had been coated with 0.1% gelatin for 30 min and contained 10% FBS-supplemented KSB-3 medium. The time point at which EB was attached was set as subculture passage 0. In addition, to compare differentiation efficiency, EBs were also attached to EGM-2MV and DMEM-F12 media, in addition to KSB-3 medium.

On day 5 after EB attachment, EBs were separated from the culture dish using TrypLE, separated into single cells by pipetting, and then attached to a new culture dish, and cultured in KSB-3 medium, which was set as subculture passage 1. Thereafter, the medium was replaced every 2-3 days, and whenever 80-90% confluency occurred by cell proliferation, 4×10⁵ cells were subcultured in a 100 mm culture dish using TrypLE and Trypan blue staining. This process was repeatedly performed to induce differentiation into MSCs (FIG. 6A). Surface antigen expression of differentiated MSCs was analyzed using FACS, and the time required for subculture was measured.

As a result, it was confirmed that homogeneous cells were stably proliferated in an experimental group using KSB-3, as compared with those in EGM-2MV or DMEM-F12 as the EB culture medium (FIG. 6B). Further, as a result of FACS analysis to examine expression of the surface antigen, the use of EGM-2MV showed low expression of CD44 and CD105, and the use of DMEM/F12 showed low expression of CD105, as compared with the use of KSB-3 as the culture medium (FIG. 6C). Further, as a result of measuring the time required for subculture, it was confirmed that more than 80 hours were taken at passage 5 in DMEM/F12 and after passage 6 in EGM-2MV, indicating delay of cell proliferation, whereas the cell proliferation rate was similar to the initial rate up to passage 11 in KSB-3 (FIG. 7).

These results indicate that KSB-3 may more efficiently differentiate induced pluripotent stem cells into mesenchymal stem cells, as compared with other media.

### Example 2-2: Examination of cell morphology of prepared iMSCs

In order to examine whether EBs were differentiated into MSCs, the cell morphology was observed through a microscope during the MSC differentiation induction process of Example 2-1.

As a result, a short fibroblast form, which is a unique morphology of MSC, was observed (FIG. 8A), indicating differentiation of iPSCs into MSCs through the process of Example 2-1.

### Example 2-3: Examination of cell proliferation potential of prepared iMSCs

In order to determine the possibility of commercialization as a cell therapy product, the number of iMSCs differentiated and proliferated from a single iPSC colony was examined.

In detail, in the process of Example 2-1, iPSCs were suspension-cultured to generate EBs, and then the number of differentiated and proliferated iMSCs according to the number of EBs was examined during subculture.

As a result, it was confirmed that a single cell separated from EB may proliferate on average 70 times (FIG. 8B), indicating that it is possible to secure a sufficient amount of cells to be used as a cell therapy product.

### Example 2-4: Examination of MSC-specific cell surface antigen expression of prepared iMSCs

In order to examine whether the iMSCs prepared in the present invention have intrinsic characteristics of MSCs, surface antigen expression was examined by FACS analysis.

In detail, the iMSCs prepared by the method of Example 2-1 were treated with seven kinds of antigens known as the surface antigens of MSCs to induce cell surface attachment, and then expression of the antigens (CD11b, CD19, CD34, CD45, HLA-DR) which are not attached to the cell surface to exhibit negative results and the antigens (CD73, CD105) which are attached to the cell surface to exhibit positive results was examined by FACS analysis (FIG. 9A). As a result of the analysis, the prepared iMSCs showed results consistent with the MSC-specific surface antigen expression pattern, indicating that differentiation of iMSCs was normally induced.

### Example 2-5: Examination of sternness of prepared iMSCs

It was confirmed whether the expression of stemness markers specifically expressed in iPSCs was lost during the differentiation of iPSCs into iMSCs prepared in the present invention.

In detail, to examine loss of stemness at the gene level, RNA was isolated from the iMSCs prepared through the process of Example 2-1, and then cDNA was synthesized, followed by real-time PCR. As a result, it was confirmed that the expression of POU5F1, SOX2, REX1, and ZNF281, which are iPSC-specific expressing genes, was significantly reduced in iMSCs (FIG. 9B).

Further, in order to examine loss of stemness at the protein level, proteins were isolated from the iMSCs prepared through the process of Example 2-1, and then the intracellular expression level of SOX2, which is a representative stemness protein, was measured by western blotting. As a result of the analysis, SOX2 protein expression was observed in iPSCs, whereas SOX2 expression was decreased in iMSCs, and thus no band was observed (FIG. 9C), indicating that stemness was lost as iPSCs differentiated into iMSCs.

### Example 2-6: Examination of differentiation potential of prepared iMSCs

MSCs have a differentiation potential for adipogenesis, osteogenesis, and chondrogenesis according to differentiation conditions, and therefore, in order to examine the differentiation potential of iMSCs prepared in Example 2-1, differentiation into adipocytes, osteocytes, and chondrocytes was induced for 4 weeks using a StemPro Adipogenesis Differentiation kit (Gibco, A10070-01), a StemPro Osteogeneis Differentiation kit (Gibco, A10072-01), and a StemPro Chnodrogenesis Differentiation kit (Gibco, A10071-01). As a result of the experiment, differentiation into osteocytes/chondrocytes/adipocytes was observed (FIG. 10A), and representatively, differentiation into osteocytes was confirmed morphologically (FIG. 10B).

These results indicate that MSCs prepared in the present invention may differentiate into adipocytes, osteocytes, and chondrocytes, and have a general MSC differentiation potential.

### Example 2-7: Examination of stability of prepared iMSCs

Karyotype analysis was performed to verify the genetic stability of the prepared iMSCs. As a result of the analysis, the normal karyotype of 46+XX was observed (FIG. 11A), indicating that the MSC differentiation method of Example 2-1 may maintain genetic stability.

In addition, in order to verify *in vivo* stability of the prepared iMSCs, the prepared iMSCs and the equal amount of iPSCs were subcutaneously injected into an experimental mouse. After 8 weeks, it was confirmed that tumors were formed at the site where iPSCs were injected into the experimental mouse, whereas no tumor was formed at the site into which iMSCs were injected (FIG. 11B). These results indicate that the iMSCs of the present invention secured stability without tumor formation, even when injected into the body.

### Example 2-8: Hierarchical clustering of genes of prepared iMSCs

For hierarchical clustering of genes of the iMSCs prepared in the present invention, microarray was performed.

In detail, hierarchical clustering of hDFs, iPSCs generated by introducing Yamanaka factors into hDFs, and iMSCs prepared by inducing differentiation of iPSCs as in Example 2-1 were compared and analyzed. As a result of the analysis, it was confirmed that hDFs, iPSCs, and iMSCs each have an independent gene expression system, and iMSCs exhibit a hierarchical clustering separated from iPSCs while having a gene expression system more similar to iPSCs than hDFs (FIGS. 11C and 11D).

### Example 3: Preparation of HLA-homo iMSCs from HLA class I homozygous (HLA-homo) iPSCs and characterization thereof

In order to prepare HLA class I homo iMSCs, EBs were generated from the HLA class I homo iPSCs prepared in Example 1, and differentiation into MSCs was induced, in the same manner as described in Example 2-1 (FIG. 12A).

In addition, to confirm whether the prepared HLA class I homo iMSCs exhibit the general characteristics of MSCs, the following experiment was performed.

In detail, as a result of observing cell morphology of the prepared HLA class I homo iMSCs in the same manner as in Example 2-2, the MSC-specific cell morphology, such as fibroblast cells, was observed (FIG. 12B).

In addition, as a result of examining expression of the MSC-specific surface antigens in the same manner as in Example 2-4, the prepared HLA class I homo iMSCs showed a result consistent with the MSC-specific surface antigen expression patterns (negative: CD11b, CD19, CD34, CD45, HLA-DR; positive: CD73, CD105) (FIG. 12C).

These results indicate that HLA class I homo iMSCs prepared by using HLA class I homo iPSCs also have the characteristics of MSCs.

Based on the above description, it will be understood by those skilled in the art that the present disclosure may be implemented in a different specific form without changing the technical spirit or essential characteristics thereof. Therefore, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds, are therefore intended to be embraced by the claims.

## Claims

1. A method of preparing mesenchymal stem cells, the method comprising the steps of:
(a) reducing an expression level of HLA class I of induced pluripotent stem cells (iPSCs) using a gene scissors technology;
(b) generating embryoid bodies (EBs) from the induced pluripotent stem cells of the step (a); and
(c) differentiating the embryoid bodies of the step (b) into mesenchymal stem cells (MSCs).

2. The method of claim 1, wherein the HLA class I is one or more selected from the group consisting of HLA-A, HLA-B, and HLA-C.

3. The method of claim 1, wherein the gene scissors technology of the step (a) is any one of CRISPR, TALEN, and zinc finger nuclease.

4. The method of claim 1, wherein the step of reducing the expression level of HLA class I of induced pluripotent stem cells (iPSCs) using the gene scissors technology in the step (a) is to use a composition for knocking out HLA class I.

5. The method of claim 4, wherein the composition for knocking out HLA class I includes one or more of (a) an expression vector including an isolated polynucleotide encoding a guide RNA consisting of a nucleotide sequence of SEQ ID NO: 1 or a nucleotide sequence complementary thereto; (b) an expression vector including an isolated polynucleotide encoding a guide RNA consisting of a nucleotide sequence of SEQ ID NO: 2 or a nucleotide sequence complementary thereto; and (c) an expression vector including an isolated polynucleotide encoding a guide RNA consisting of a nucleotide sequence of SEQ ID NO: 3 or a nucleotide sequence complementary thereto.

6. The method of claim 5, wherein the expression vector includes a polynucleotide encoding Cas9 nuclease, Cpf1 nuclease, or Cas9 nickase.

7. A transplantation-compatible mesenchymal stem cell prepared by any one method of claims 1 to 6.

8. A pharmaceutical composition for preventing or treating any one disease selected from the group consisting of immune diseases, inflammatory diseases, neurological diseases, bone diseases, and arthropathy, the pharmaceutical composition comprising the transplantation-compatible mesenchymal stem cell of claim 7 as an active ingredient.

9. The pharmaceutical composition of claim 8, wherein the disease is osteoporosis, rheumatoid arthritis, or a periodontal disease.

10. A cell therapy product comprising the transplantation-compatible mesenchymal stem cell of claim 8 as an active ingredient.

11. A method of preparing transplantation-compatible induced pluripotent stem cells, the method comprising the step of reducing an expression level of HLA class I using a gene scissors technology.

12. A transplantation-compatible induced pluripotent stem cell prepared according to the method of claim 11.

13. A method of preparing embryonic stem cells (ESCs), the method comprising the step of reducing an expression level of HLA class I using a gene scissors technology.

14. A transplantation-compatible embryonic stem cell prepared according to the method of claim 13.
